# EUROPEAN PATENT APPLICATION

(11) **EP 2 799 065 A1**
(43) Date of publication of application: **05.11.2014**
(21) Application number: 13165904.7
(22) Date of filing: 30.04.2013
(51) Int. Cl.: A61K 9/28, A61K 9/16, A61K 9/20, A61K 31/195, A61K 31/198, A61K 31/275

(54) **Levodopa carbidopa entacapone pharmaceutical formulations**

(71) Applicant: Deva Holding Anonim Sirketi, 34303 Istanbul (TR)
(72) Inventor: Koc, Fikret, 34303 Istanbul (TR); Kandemir, Levent, 34303 Istanbul (TR); Aldeniz, Emre, 34303 Istanbul (TR); Ilhan, Suna Ayse, 34303 Istanbul (TR)

(57) **Abstract**

The present invention relates to a new pharmaceutical composition of entacapone, levodopa and carbidopa or pharmaceutically acceptable or hydrates thereof characterized in that characterized in that it comprises; a) a first granule wherein 90 % by weight of the total amount of levodopa is mixed with 90 % by weight of the total amount of carbidopa and 10% by weight of the total amount of entacapone, optionally in the presence of pharmaceutical acceptable excipients. b) a second granule wherein 10 % by weight of the total amount of levodopa is mixed with 10 % by weight of the total amount of carbidopa and 90% by weight of the total amount of entacapone, optionally in the presence of pharmaceutical acceptable excipients. c) Optionally mixed other pharmaceutically acceptable excipients, d) coating of core tablets is composed of hydroxypropyl cellulose and hydroxypropyl methyl cellulose.

## Description

### Technical Field

The present invention relates to a new pharmaceutical composition of entacapone, levodopa and carbidopa or pharmaceutically acceptable salts or hydrates thereof characterized in that characterized in that it comprises; a) a first granule wherein 90 % by weight of the total amount of levodopa is mixed with 90 % by weight of the total amount of carbidopa and 10% by weight of the total amount of entacapone, optionally in the presence of pharmaceutical acceptable excipients. b) a second granule wherein 10 % by weight of the total amount of levodopa is mixed with 10 % by weight of the total amount of carbidopa and 90% by weight of the total amount of entacapone, optionally in the presence of pharmaceutical acceptable excipients. c) Optionally a third mixture of pharmaceutically acceptable excipients, d) coating of core tablets is composed of hydroxypropyl cellulose and hydroxypropyl methyl cellulose.

### Background Art

Parkinson is a degenerative disorder of the central nervous system and is characterized by progressive tremor, rigidity, brady kinesia, disturbances of posture balance and gait. There is no cure for Parkinson's disease, but medications can provide relief from the symptoms.

Parkinson is usually treated with levodopa, dopamine agonist, MAO-B inhibitors, dopa-decarboxylase inhibitor.

Levodopa (3, 4-dihydroxyphenylalanine) or L-DOPA is an immediate precursor of dopamine. Parkinson's disease patients often take between 200 mg and 2 g of levodopa per day with late stage Parkinson's patients taking toward the later end of this range. Monotherapy with levodopa is often accompanied by unpleasant side effects such as nausea and vomiting.

Administration of combination of levodopa and a dopa decarboxylase inhibitor such as carbidopa decreases patient's side effects while increasing drug efficacy.

Carbidopa is a peripheral dopa-decarboxylase inhibitor (DCC) which exhibits a distinct pharmacological activity only when given in combination with levodopa. Carbidopa inhibits the peripheral decarboxylation of levodopa to dopamine in the systemic circulation, allowing for greater levodopa distribution into the central nervous system.

Entacapone is chemically, (2*E*-)-2-cyano-3-(3, 4-dihydroxy-5-nitrophenyl)-N, N-diethylprop-2-enamide.It corresponds to the molecular formula C₁₄H₁₅N₃O₅ and relative molecular mass of 305.29.

US 5446194 B (ORION YHTYMA OY) 29.08.1995 discloses entacapone as a cathecol-o-methyl transferase (COMT) inhibitor.

Entacapone is catechol-o-methyltransferase (COMT) inhibitor, is nitro catechol structured compound used in the treatment of Parkinson's disease. Entacapone is prescribed as an adjunct of L-dopa therapy in parkinson disease and clinically used in levodopa/dopa decarboxylase inhibitor therapy who are experiencing motor fluctuations. This extends the effect and duration of levodopa in the brain and allows levodopa to be given less often and in lower doses.

Entacapone is a class IV drug under the Biopharmaceutics Classification System and poses problems of low solubility and low dissolution rate and low bioavailability quickly absorbed in the gastrointestinal tract.

It appears as yellow non-hygroscopic crystalline powder, practically insoluble in water, soluble or sparingly soluble in acetone, slightly soluble in anhydrous ethanol.

The bioavailability of entacapone after oral administration is low and subject to large interindividual variation. Methods of increase the bioavailability by improving its solubility and dissolution properties are disclosed in various patent applications.

EP 1112065 A (ORION CORPORATION) 17.12.2003 discloses the use of cross linked cellulose derivative as a dissolution enhancing agent in an oral compacted entacapone composition.

WO 2009/098661 (WOCKHARDT RESEARCH CENTER) 13.08.2009 discloses single oral dose pharmaceutical compositions comprising a combination of entacapone, levodopa and carbidopa, or salts thereof along with one or more sugar alcohols, wherein the entacapone is co-micronized with one or more sugar alcohols.

Combination of levodopa, carbidopa and entacapone is currently available in dose formulations of 50 mg/12.5 mg/200 mg, 100 mg/25 mg/200 mg, 150 mg/37.5 mg/200 mg and is marketed as STALEVO. Entacapone/levodopa/carbidopa combination tablet is indicated for the treatment of patients with idiopathic Parkinson's disease who experience signs and symptoms of end-of-dose 'wearing off'.

Various formulations corresponding to combination of entacapone, levodopa and carbidopa have been disclosed in the literature.

WO 2008/053297 (WOCKHARDT LIMITED) discloses formulations where entacapone is separated from levodopa and carbidopa.

EP 1189608 B (ORION CORPORATION) 23.07.2003 discloses a pharmaceutical composition comprising entacapone, levodopa, and carbidopa or pharmaceutically acceptable salts or hydrates thereof wherein carbidopa is added separately to the composition e.g. entacapone and levodopa, together with a(n) excipient(s).

### Summary of invention

The present invention relates to a new pharmaceutical composition of entacapone, levodopa and carbidopa or pharmaceutically acceptable salts or hydrates thereof characterized in that it comprises; a) a first granule wherein 90 % by weight of the total amount of levodopa is mixed with 90 % by weight of the total amount of carbidopa and 10% by weight of the total amount of entacapone, optionally in the presence of pharmaceutical acceptable excipients. b) a second granule wherein 10 % by weight of the total amount of levodopa is mixed with 10 % by weight of the total amount of carbidopa and 90% by weight of the total amount of entacapone, optionally in the presence of pharmaceutical acceptable excipients. c) Optionally a third mixture of pharmaceutically acceptable excipients, d) coating of core tablets is composed of hydroxypropyl cellulose and hydroxypropyl methyl cellulose.

### Technical Problem

Nowadays, fixed dose combinations of drugs are more preferable in an effort to improve patient adherence and convenience, reduce cost and improve treatment efficacy and reduce resistance. But, compounds' absorptions from the digestive tract could be highly variable and it could be very difficult to achieve comparative in vitro dissolution profiles for different active agents in an oral solid fixed dose composition.

Fixed combination products require enabling formulations to ensure correct dissolution profiles for all active pharmaceutical ingredients.

Accordingly, chemical and physical characteristics of active ingredients have to be considered and related problems have to be challenged by formulation scientist to reach a non problematic and stabile product.

In pharmaceutical manufacturing, granulation methods are widely used due to consistent and high quality granular product in fixed dose combinations. Selection of the correct granulation technique and ingredients are mainly based on active ingredients and desired product characteristics. One method of making granules is wet granulation and it is frequently used and involves steps such as milling, dry mixing, wet massing, drying and milling of powder.

Therefore, combination formulation of levodopa, carbidopa and entacapone in a single unit formulation is intriguing in consideration of achieving target dissolution profile in all active ingredients. Meantime, minimizing carbidopa's degradation products is mainly aimed across formulations by scientists.

Carbidopa is slightly soluble in water but dissolve readily in dilute minerals and it is used as monohydrate which exists in a stable crystal form.

Although carbidopa is stable as the bulk drug substance, it is a relatively fragile molecule and formulations containing often show relatively poor conservation, particularly at high temperature and high humidity. It is known to degrade readily in solution to form known and unknown impurities which are also observed as the primary degradation products in the finished tablet.

Carbidopa may undergo Michael addition to entacapone to form additional degradation products. Formulation scientist aims to minimize the existing degradation via the tablet manufacturing process.

Although water is commonly used as granulation solvent for economical and ecological reasons, stability of carbidopa is adversely affected by water; causing hydrolysis of susceptible products.

In formulations containing carbidopa and acceptable salts thereof, correct selection of appropriate granulation solvent, and drying process are extremely important. Stability is affected due to the extended exposure to heat.

Accordingly, the excessive heating during drying may cause degradation of product or impact significantly the properties and thus functionality of the product.

Therefore, there is a need for developing pharmaceutical compositions which incorporate levodopa, carbidopa and entacapone or their pharmaceutical acceptable salts and methods for preparing such compositions with an improved physical stability and without the active ingredient release properties being affected and efficient and industrial feasible methods for their preparation.

### Solution to Problem

A simple and reproducible formulation and pharmaceutical manufacturing method for oral solid dosage forms of combination of levodopa, carbidopa and entacapone are aimed to be developed by the present invention to overcome problems that are encountered in manufacturing.

The inventors of the present invention are aimed to develop a pharmaceutical formulation containing levodopa, carbidopa and entacapone or a pharmaceutically acceptable salts or hydrates thereof having a good dissolution profile and an improved physical stability without affecting the release profile of any active ingredients.

When inventors of this application attempt to develop a fixed single solid dosage form containing entacapone, levodopa and carbidopa or pharmaceutically acceptable salts or hydrates thereof, many difficulties were broken out due to physical characteristics of active ingredients, namely carbidopa and entacapone.

In this invention, inventors are undertaken investigations on both developments of different pharmaceutical formulations and manufacturing methods. They have developed a formulation of drug where entacapone, levodopa and carbidopa or their pharmaceutically acceptable salts or hydrates are combined into an oral solid composition.

Inventors of this invention have surprisingly found that encountered difficulties in the formulation can be overcome by preparation of two separate granules comprising entacapone, levodopa and carbidopa or pharmaceutically acceptable salts or hydrates thereof.

This invention is directed to a) a first granule wherein 90 % by weight of the total amount of levodopa is mixed with 90 % by weight of the total amount of carbidopa and 10% by weight of the total amount of entacapone, optionally in the presence of pharmaceutical acceptable excipients; b) a second granule wherein 10 % by weight of the total amount of levodopa is mixed with 10 % by weight of the total amount of carbidopa and 90% by weight of the total amount of entacapone, optionally in the presence of pharmaceutical acceptable excipients; c) Optionally mixed other pharmaceutically acceptable excipients d) coating of core tablets is composed of hydroxypropyl cellulose and hydroxypropyl methyl cellulose.

The ratios of levodopa, carbidopa and entacapone are arranged and adjusted in both granules to achieve correct dissolution profile of original product and presented formulations surpass previous problems arising when formulating the combination of entacapone, levodopa and carbidopa or pharmaceutically acceptable or hydrates thereof.

### Description of embodiments

In the present invention, the present inventors have aimed to provide pharmaceutical compositions containing levodopa, carbidopa and entacapone or pharmaceutically acceptable salts or hydrates thereof for oral administration having a good dissolution profile and an improved physical stability without affecting the release profile of said active ingredients.

In the present invention, "entacapone" is understood to be the compound entacapone or a pharmaceutically acceptable salt, hydrate o solvate thereof. Entacapone may be incorporated in the composition in any solid form either as a free compound or as a hydrate or solvate.

Unless otherwise indicated, as "levodopa" and "carbidopa" it is understood the compounds levodopa and carbidopa or their pharmaceutically acceptable salts, hydrates or solvates. Carbidopa is preferably used in form of monohydrate.

In the first aspect of the invention, the present inventors have aimed to improve carbidopa' s stability and performed different manufacturing process, especially wet granulations by using different solvents. Granulations of carbidopa are evaluated and critical parameters have been determined for the process.

Inventor's excessive studies have revealed that carbidopa intimately mixed with water is more prone to degradation and basic feature of the water solution activates changes to drug resulting degradation of carbidopa.

On the other hand, the drying process can impact properties, namely functionality and quality of granules being dried. Therefore, it is necessary to optimize the drying time to ensure the good quality of the product.

High drying temperature has been identified as high risk factor for water granulated carbidopa, particularly considering the fact that longer drying time is needed higher drying temperature.

On these grounds, inventors of the present invention have determined anhydrous ethanol as granulation solvent. The latter reduces drying time for making granules which provides a shorter and more reproducible process.

Inventors have defined a specific range for using anhydrous ethanol. And quantity is set in order to minimize by reasons of safety and quality purposes.

The chemical stability of carbidopa is provided by avoiding basic medium in the present invention.

Simple and reproducible formulations and techniques are developed to minimize production site and related batch to batch manufacturing problems.

Presented formulations inhibit degradation of carbidopa during prolonged storage under ambient conditions. Embodiments of this invention enable formulations with degrading levels 0.13 % for unknown impurities.

In a second aspect of the present invention, the wet granulated formulation containing levodopa, carbidopa and entacapone or pharmaceutically acceptable salts or hydrates thereof is then dried using tray oven to reduce water content to a predetermined level. Drying can be conducted at various temperatures and times. Tray drying ovens are especially preferred as volatile organic solvent can evaporate and dry more quickly.

In another aspect of the present invention, two different granules where levodopa carbidopa and entacapone are in admixture are prepared to achieve comparative in vitro dissolution profiles.

Accordingly, another aspect of this invention is a solid pharmaceutical composition for oral administration comprising entacapone, levodopa and carbidopa or pharmaceutically acceptable salts or hydrates thereof characterized in that it comprises;

a) a first granule wherein 90 % by weight of the total amount of levodopa is mixed with 90 % by weight of the total amount of carbidopa and 10% by weight of the total amount of entacapone, optionally in the presence of pharmaceutical acceptable excipients.

b) a second granule wherein 10 % by weight of the total amount of levodopa is mixed with 10 % by weight of the total amount of carbidopa and 90% by weight of the total amount of entacapone, optionally in the presence of pharmaceutical acceptable excipients.) optionally a third mixture of pharmaceutically acceptable excipients; d) coating of core tablets is composed of hydroxypropyl cellulose and hydroxypropyl methyl cellulose.

Then the two prepared granules are mixed with other excipients, pressed and coated with a coating.

In another aspect of the present invention, entacapone 's quick absorption from the gastrointestinal tract is controlled via an appropriate formulation. Quickly absorbed than levodopa and carbidopa, entacapone dissolution is a main challenge of formulation scientist and needed to be adjusted when formulating.

The inventors have scoped out the occurring problem and aimed to adjust entacapone dissolution in combination with levodopa and carbidopa.

Traditionally, immediate release film coatings have relatively simple formulations, such as combining a single polymer in combination with plasticizer and colorants and Hydroxypropylmethyl cellulose (HPMC) is known for its good strip-forming properties and has excellent acceptability.

In order to slow down entacapone dissolution profile, several types of hydrophilic polymers have been investigated and it is concluded that only use of combination of HPC and HPMC is useful in controlling the release of entacapone.

In another aspect, the unit dosage form has levodopa, carbidopa and entacapone or pharmaceutical acceptable salt and one or more pharmaceutically acceptable excipients as components. With a tablet dosage form the one or more excipients can be chosen from disintegrates, binders, diluents, glidants, lubricants, colouring agents, stabilizers, preservatives, and/or flavoring agents.

The term 'filler' and the term 'diluent' are herein used interchangeably. Fillers fill out the size of a composition, making it practical to produce and convenient for the consumer to use. Suitable filler/diluent includes, but are not limited, to calcium carbonate, calcium phosphate, dibasic calcium phosphate, tribasic calcium sulphate, calcium carboxymethylcellulose, cellulose, dextrin derivatives, dextrin, dextrose, fructose, lactitol, lactose lactose (e.g. spray-dried lactose, a-lactose, β-lactose, Tablettose®, various grades of Pharmatose®, Microtose® or Fast-Floc®), methylcellulose polymers such as, e.g., Methocel A®, Methocel A4C®, Methocel A 15C®, Metocel A4M®), hydroxyethylcellulose, hydroxypropylcellulose, L-hydroxypropylycellulose (low substituted), hydroxypropyl methylcellulose (HPMC) (e.g. Methocel E®, F and K, Metolose SH® of Shin-Etsu, grades of Methocel F® and Metolose 65 SH®, the 4,000, 15,000 and 100,000 cps grades of Methocel K®; and the 4,000, 15,000, 39,000 and 100,000 grades of Metolose 90 SH®), sodium carboxymethylcellulose, carboxymethylene, carboxymethylhydroxyethylcellulose and other cellulose derivatives, starches or modified starches ( including potato starch, wheat starch, corn starch, rice starch, pregelatinized maize starch), magnesium carbonate, magnesium oxide, maltitol, maltodextrins, maltose, sorbitol, starch, sucrose, sugar, and xylitol, erythritol.

A binder is used to impart cohesive qualities to the solid dosage form and thus ensure that a tablet remains intact after compression. Examples of binders include, but not limited to, microcrystalline cellulose, hydroxymethyl cellulose, hydroxypropylcellulose, starch (including corn starch and pregelatinized starch), gelatin, sugars (including sucrose, glucose, dextrose, lactose, and sorbitol), waxes, polyethylene glycol, natural and synthetic gums (e.g.acacia, tragacanth sodium alginate, celluloses, and Veegum),and synthetic polymers such as polymetacrylates and polyvinylpyrrolidone (povidone), ethylcellulose, hydroxyethyl cellulose, polyethylene oxide, mixtures thereof and the like.

A disintegrant is a substance which helps the composition break up once ingested .Disintegrants are, but not limited to, cross linked polyvinylpyrolidone (crospovidone, polyplyplasdone XL®, kollidon CL®); starches such as maize starch and dried sodium starch glycolate; gums such as maize starch and dried sodium starch glycolate; gums such as alginic acid, sodium alginate, guar gum; croscarmellose sodium; cellulose products such as microcrystalline cellulose and its salts, micro fine cellulose, low-substituted hydroxypropylcellulose, mixtures thereof and the like.

Glidants improve the flowability of the composition. The composition may also comprise a glidant. Glidants are, but not limited to, colloidal silicon dioxide, calcium silicate, magnesium trisilicate, powdered cellulose, talc, tribasic calcium phosphate, mixtures thereof and the like.

The glidant is preferably present in 0.1% to 5% by weight based on the total mass of the pharmaceutical composition, more preferably from 0.5% to 4%, most preferably from 0.5 % to 3%,

The presence of a lubricant is particularly preferred when the composition is a tablet as lubricants to improve the tableting process. Lubricants prevent composition ingredients from clumping together and from sticking to the tablet punches or capsule filling machine and improve flowability of the composition mixture. Lubricants are, but not limited to sodium oleate, sodium stearate, sodium benzoate, sodium stearate, sodium chloride, stearic acid, sodium stearyl fumarate, calcium stearate, magnesium stearate, magnesium lauryl sulfate, sodium stearyl fumarate, sucrose esters or fatty acid, zinc, polyethylene glycol, talc, mixtures thereof and the like.

Coating agents are, but not limited to, ethyl cellulose ,methyl cellulose, hydroxypropyl-methyl cellulose, hydroxyethyl cellulose, hydroxypropyl cellulose,polyvinylalcohol, polyvinyl acetate , cellulose acetate ,cellulose acetate phthalate, methylcellulose phthalate, hydroxymethyl cellulose phthalate, hydroxypropylmethylcellulose phthalate, hydroxypropylmethylcellulose succinate, cellulose acetate butyrate, methacrylate copolymers ,polymethacrylates, acetyltributyl citrate, erythritol, glyceryl palmitostearate, tributyl citrate, triethyl citrate, glyceryl monostearate, glycerin monostearate, gelatin, hydrogenated soybean oil, glyceryl monostearate,Hydrogenated oil, carboxymethyl cellulose,sodium carboxymethyl cellulose, sodium carboxymethyl starch, crosscarmellose sodium, aminoalkyl methacrylate copolymer E, low-substituted hydroxypropyl ether of cellulose,polyvinyl pyrollidone,polyethylene glycol,cellulose trimellitate, hydroxymethyl cellulose acetate succinate, resins like shellac, methylmethacrylate-methacrylic acid , methacrylic acid-ethyl acrylate,zein, hydroxyethyl-ethylcellulose phthalate, cellulose aceto succinate, polylactic acid,polyglycolic acid, gelatinized starch, ammonium hydroxide,sodium hydroxide, potassium hydroxide, magnesium hydroxide, calcium hydroxide, gums, liposomes, cyclodextrins, chitosan, gliadin,hordein,prolamine,sucrose, sorbitol, glycerin,triethylamine, diethylamine, trimethylamine, methylamine, dimethylamine, isopropylamine, triethanolamine, diethanolamine, disodium monohydrogen phosphate, dipotassium monohydrogen phosphate, mixtures thereof and the like .

Other ingredients such as colorants and titanium dioxide can also be used.

In another aspect of the present invention, a process for preparing oral solid pharmaceutical compositions, by means of a process that can be applied at an industrial level with low energy costs and which does not subject the active ingredient to aggressive formulation conditions which can entail the loss of stability of the product.

In another aspect of the present invention, organic solvent is used as granulation solvent. They are especially preferred for moisture sensitive products as they are volatile and they evaporate quickly during drying process.

Residual solvent presence in a final product is undesirable and should be avoidable. Safe and effective methods have to be used to remove them. It is impossible to completely remove all traces of the organic solvent or alcohol from the granulated drug substance. Trace solvents can leave adverse odours in the finished dosage form that are objectionable to many patients.

The solvent used in granulation process is generally a volatile hydrocarbon or alcohol which can easily be removed from the granules after they are formed. However, the use of highly volatile solvents presents a new set of problems. These solvents are generally environmentally deleterious and have a great risk of explosion during handling them.

Regulation has a strict limitation of the solvent residue in some granule products, such as in pharmaceuticals and food products. Published pharmacopeias and ICH guidelines determine maximum allowable amounts of residual solvent in pharmaceutical products.

The following examples are given for the purpose of illustration of the present invention and should not limit the scope of the invention.

### Example 1 (Core Tablet Unit Formula)

**Table 1**

| **Ingredients** | **mg/tablet** |
|---|---|
| Levodopa | 200.0 |
| Carbidopa Monohydrate | 54.0 |
| Entacapone | 200.0 |
| Microcrystalline cellulose | 79.0 |
| Maltodextrin | 160.0 |
| Croscarmellose sodium | 28.0 |
| Hydroxypropylmethyl cellulose | 15.0 |
| Colloidal silicon dioxide | 7.0 |
| Mg Stearate | 7.0 |
| **Core tablet weight** | **750.0** |

### Example 2 Manufacturing Method)

Pharmaceutical formulation may be prepared using methods as below without limiting scope of this invention;

1. Weigh 90% of Levodopa, 90%Carbidopa monohydrate and 10% of entacapone.

2. Sieve powder mixture in Step 1 through an appropriate sieve.

3. Weigh hydroxypropylmethyl cellulose, 1/3 of maltodextrin and 33% of croscarmellose sodium and sieve them through an appropriate sieve.

4. Add these materials into the mixture obtained in step 2 and blend.

5. Granulate the powder mixture obtained in step 3 with ethanol anhydrous.

6. Dry and sieve granules obtained in step 5. Let this be Granula 1.

7. Weigh 10% of Levodopa, 10%Carbidopa monohydrate and 90% of entacapone.

8. Sieve powder mixture in Step 7 through an appropriate sieve.

9. Weigh microcrystalline cellulose, povidone, 1/3 of maltodextrin and 33% of croscarmellose sodium and sieve them through an appropriate sieve.

10. Add these materials into the mixture obtained in step 9 and blend.

11. Granulate the powder mixture obtained in step 10 with ethanol anhydrous.

12. Dry and sieve granules obtained in step 11. Let this be Granula 2.

13. Mix Granula 1 and Granula 2.

14. Weigh rest of maltodextrin, croscarmellose sodium and colloidal silicon dioxide.

15. Sieve powder mixture in Step 14 through an appropriate sieve. And add into the mixture obtained in step 13.

16. Blend the mixture obtained in step 15.

17. Sieve magnesium stearate and add this into the mixture obtained in step and blend.

18. Compress the powder obtained in step 17 into tablets.

19. Coat the tablets with coating material until desired weight is gained.

### Example 3(Coating Formula)

**Table 2**

| **Coating Ingredients** | **%(w/w)** |
|---|---|
| HPMC E3 | 10 |
| HPMC E15 | 25 |
| HPC | 25 |
| Red iron oxide | 20 |
| Talc | 5 |
| Glycerin | 10 |
| Titanium Dioxide | 5 |

## Claims

1. An oral solid composition comprising pharmacologically effective amounts of entacapone, levodopa and carbidopa, or pharmaceutically acceptable salts or hydrates thereof, and pharmaceutically acceptable excipients **characterized in that** it comprises a) a first granule wherein 90 % by weight of the total amount of levodopa is mixed with 90 % by weight of the total amount of carbidopa and 10% by weight of the total amount of entacapone, optionally in the presence of pharmaceutical acceptable excipients. b) a second granule wherein 10 % by weight of the total amount of levodopa is mixed with 10 % by weight of the total amount of carbidopa and 90% by weight of the total amount of entacapone, optionally in the presence of pharmaceutical acceptable excipients. c) Optionally mixed other pharmaceutically acceptable excipients, d) coating of core tablets is composed of hydroxypropyl cellulose and hydroxypropyl methyl cellulose.

2. In preparing of granules according to claim 1, anhydrous ethanol is used as a solvent.
